(19) Europäisches Patentamt
European Patent Office
Office européen des brevets

(11) **EP 3 981 386 A1**

(12) **EUROPEAN PATENT APPLICATION**
published in accordance with Art. 153(4) EPC

(43) Date of publication:
**13.04.2022 Bulletin 2022/15**

(21) Application number: **20819260.9**

(22) Date of filing: **06.05.2020**

(51) International Patent Classification (IPC):
**A61K 8/49** $^{(2006.01)}$    **A61Q 17/04** $^{(2006.01)}$

(52) Cooperative Patent Classification (CPC):
**A61K 8/49; A61Q 17/04**

(86) International application number:
**PCT/KR2020/005969**

(87) International publication number:
**WO 2020/246715 (10.12.2020 Gazette 2020/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.06.2019 KR 20190066104**

(71) Applicant: **Ulsan National Institute of Science and
Technology
(UNIST)
Eonyang-eup Ulju-gun
Ulsan 44919 (KR)**

(72) Inventors:
• **JANG, Ji Wook**
  **Ulju-gun Ulsan 44919 (KR)**
• **LEE, Jae Sung**
  **Ulju-gun Ulsan 44919 (KR)**
• **BYUN, Woo Jin**
  **Ulju-gun Ulsan 44919 (KR)**

(74) Representative: **Calysta NV
Lambroekstraat 5a
1831 Diegem (BE)**

(54) **ULTRAVIOLET ABSORBER, PREPARATION METHOD THEREFOR, AND ULTRAVIOLET SCREENING PRODUCT CONTAINING SAME**

(57) The present disclosure relates to an ultraviolet absorber, a preparation method therefor, and an ultraviolet screening product containing same and, more specifically, to: an ultraviolet absorber, which comprises polymeric carbon nitrides having a heterocyclic structure comprising C and N; a preparation method therefor; and an ultraviolet screening product containing same.

FIG. 1

**EP 3 981 386 A1**

## Description

## Technical Field

The following description relates to an ultraviolet absorber, a method for preparing the same, and an ultraviolet blocking product containing the same.

Background Art

Excessive exposure to ultraviolet (UV) radiation coming out of the sun is said to cause skin cancer. Therefore, a sunscreen should be used in order to prevent life-threatening diseases and wrinkles on the skin. Most sunscreens consist of organic sunscreens, but due to the lack of eco-friendliness, there have recently been such things as the prohibition of use of organic sunscreens in Hawaii from 2021, and products composed mainly of inorganic sunscreens have been attracting attention. However, the inorganic sunscreens also have some side effects. Two frequently used inorganic sunscreens are zinc oxide (ZnO) and titanium dioxide ($TiO_2$), but the use of these inorganic sunscreens causes serious health problems. Although a metal oxide semiconductor sun cream with such photoactivity is a complete UV spectrum blocker, it produces a hydroxyl group ($\cdot OH$) and a peroxide group ($O_2 \cdot -$) in this process. The production of these highly reactive oxygen species (ROS) not only decomposes the organic additives of sunscreen, but also induces oxidative stress in the skin tissue, causing damage at the cellular level and promoting DNA modification and inflammatory response. After all, another carcinogen is produced while trying to protect the skin. Besides, the use of micro-sized ZnO and $TiO_2$ in the sunscreen drops the ultraviolet blocking efficiency and also causes unfavorable white turbidity in cosmetics. However, when nanoparticles are used in order to improve aesthetic parts, more ROS is generated, and the possibility that the nanoparticles penetrate into skin cells is increased so that health risks are more increased.

Organic sunscreens are much more serious than inorganic sunscreens. Organic sunscreens such as avobenzone, oxybenzone, oxylmethox, octyl methoxynemanate, etc. easily penetrate deep into the skin, accumulate in hair follicles, and flow in the bloodstream or other body fluids, causing hormonal disturbances and cell damage. In addition, several recent studies highlight the negative effects of these organic sunscreens on male reproduction, sperm quality, and sperm function. According to them, the accumulation of these cosmetics may damage male fertility. They also highlight the detrimental effects of organic sunscreens on the female hormone progesterone. Organic sunscreens not only have a bad effect on the human body, but also cast a long shadow on the ecosystem. These many sunscreen additives are decomposed into harmful chemicals when they come in contact with chlorinated water or seawater under ultraviolet light.

Recently, an increasing number of studies are attempting to remove carcinogenic ROS by a method of encapsulating the above inorganic and organic substances, but their potential for commercialization has not yet been proven. The only method capable of solving such a life-threatening problem is to synthesize a UV filter which absorbs the entire UV spectra, is very stable, has low photocatalytic activity, has biocompatibility, adheres well to the skin, and is non-toxic. However, it is difficult to find high-efficiency integrated materials for using sunscreens.

## Disclosure of the Invention

## Technical Goals

The present disclosure is to solve the above problems, and an aspect provides a polymeric carbon nitride (PCN)-based ultraviolet absorber, which is cheap, stable and eco-friendly, has biocompatibility, and enables skin protection for the entire ultraviolet spectra (320-400 nm UVA + 290-320 nm UVB wavelength), a method for preparing the same, and an ultraviolet blocking product containing the same.

However, the problems to be solved by the present disclosure are not limited to those mentioned above, and other problems not mentioned will be clearly understood by those skilled in the art from the following description.

## Technical Solutions

According to an aspect, there is provided an ultraviolet absorber according to an example embodiment of the present disclosure, the ultraviolet absorber including polymeric carbon nitride having a heterocyclic structure containing C and N.

According to an aspect, the heterocyclic structure containing C and N may include a triazine structure, a heptazine ring structure, or a combination thereof.

According to an aspect, the ultraviolet absorber may have a sun protection factor (SPF) of 3.5 or more, and the ultraviolet absorber may have a sun protection factor (SFA) of 3.0 or more.

According to an aspect, when performing a rhodamine B decomposition reaction using the ultraviolet absorber, Equation 1 below may be satisfied.

$$[\text{Equation 1}]$$

$$C/C_0 > 0.8$$

(However, $C_0$ is the initial concentration of rhodamine B, and C is the concentration of rhodamine B remaining after the decomposition reaction.)

According to another aspect, there is provided a method for preparing an ultraviolet absorber according to an example embodiment of the present disclosure, the method including the steps of: preparing at least one precursor selected from the group consisting of melamine, dicyandiamide, cyanamide, urea, cyanuric acid, and complexes thereof; heating the precursor; drying the heated precursor; acid-treating, heat-treating, or sonicating the dried precursor; and drying the acid-treated, heat-treated or sonicated precursor.

According to an aspect, the step of heating the precursor may be performed at a temperature condition of 400°C to 600°C for 1 to 10 hours.

According to an aspect, the step of drying the heated precursor may be performed in an oven at a temperature condition of 60°C to 80°C for 8 to 24 hours.

According to an aspect, the step of acid-treating the dried precursor may be pouring $H_2SO_4$ (98%), $HNO_3$ (70%), or HCl (35%) into the dried precursor to perform acid treatment, and then pouring distilled water to generate heat.

According to an aspect, the step of heat-treating the dried precursor may be performed at a temperature condition of 400°C to 600°C for 1 to 2 hours.

According to an aspect, the step of sonicating the dried precursor may be pouring isopropyl alcohol (IPA) into the dried precursor to perform sonication for 1 to 6 hours.

According to an aspect, the step of drying the acid-treated, heat-treated, or sonicated precursor may be performed in an oven at a temperature condition of 60°C to 80°C for 8 to 16 hours.

According to an aspect, the ultraviolet absorber may be an ultraviolet absorber according to an example embodiment of the present disclosure.

According to another aspect, there is provided an ultraviolet blocking product according to an example embodiment of the present disclosure containing: an ultraviolet absorber according to an example embodiment of the present disclosure; or an ultraviolet absorber prepared through a method for preparing an ultraviolet absorber according to an example embodiment of the present disclosure.

According to an aspect, the ultraviolet blocking product may be an ultraviolet blocking cosmetic product or an ultraviolet blocking filter.

## Advantageous Effects

Since the polymeric carbon nitride (PCN)-based ultraviolet absorber according to the present disclosure has many promising features including high optical and chemical stabilities, and non-allergic properties, and contains only biologically compatible C, H, and N as chemical constituents, it is inexpensive, stable, and eco-friendly, has biocompatibility, and may implement skin protection for the entire ultraviolet spectra (320-400 nm UVA + 290-320 nm UVB wavelength).

Unlike conventional organic sunscreens, which are expensive and also have to be reapplied relatively frequently due to their low photostability, ultraviolet blocking cosmetics to which the polymeric carbon nitride (PCN)-based ultraviolet absorber according to the present disclosure is applied are inexpensive, may be simply produced, and are even stable so that they are very effective in terms of economic feasibility.

Further, unlike conventional inorganic sunscreens having high photocatalytic activities, the ultraviolet blocking cosmetics to which the polymeric carbon nitride (PCN)-based ultraviolet absorber according to the present disclosure is applied have low photocatalytic activities so that they may realize excellent light absorption ability in both UV-A and UV-B regions while making less highly reactive oxygen species.

Brief Description of Drawings

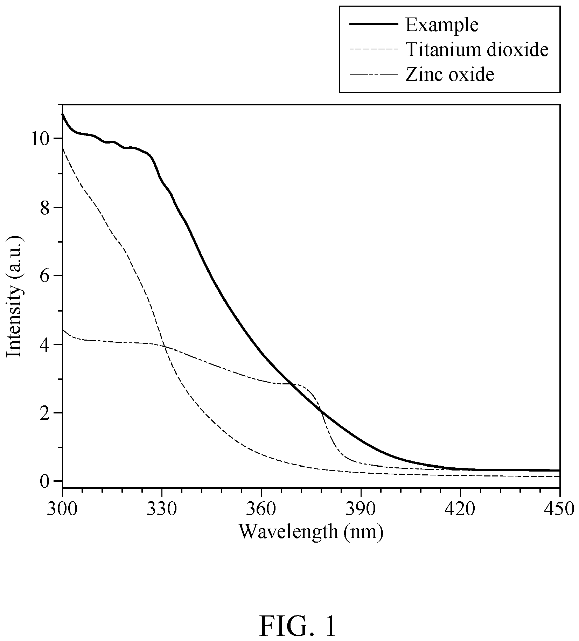

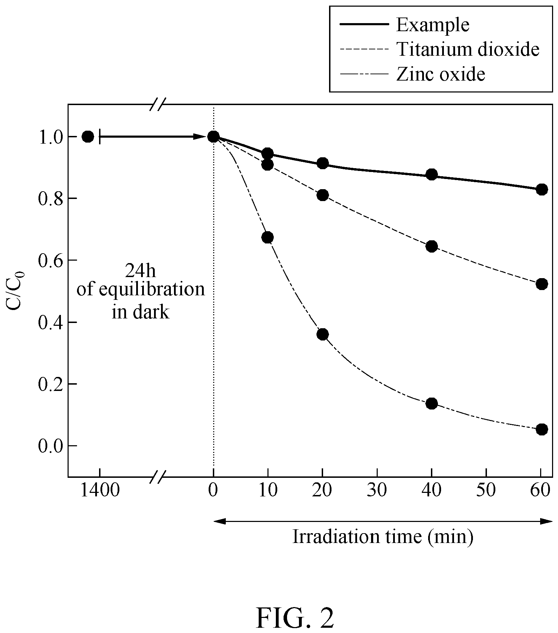

FIG. 1 is ultraviolet-visible (UV-Vis) absorption spectra of polymeric carbon nitride produced according to an example embodiment of the present disclosure. FIG. 2 is a graph comparing photocatalytic activities of polymeric carbon nitride produced according to an example embodiment of the present disclosure and conventional titanium dioxide and zinc oxide.

Best Mode for Carrying Out the Invention

Hereinafter, example embodiments of the present disclosure will be described in detail with reference to the accompanying drawings. In describing the present disclosure, if it is determined that a detailed description of a related well-known function or configuration may unnecessarily obscure the gist of the present disclosure, the detailed description thereof will be omitted. Also, terms used in the present specification, as terms which are used so as to appropriately describe a preferred embodiment of the present disclosure, may be changed depending on the user's or operator's intention or the practices of the field to which the present disclosure pertains to. Therefore, the definitions of the terms should be made based on the contents throughout the present specification. The same reference numerals disclosed in each drawing represent the same members.

In the whole specification, when any member is positioned "on" the other member, this not only includes a case that the any member is brought into contact with the other member, but also includes a case that another member exists between two members.

In the whole specification, if a prescribed part "includes" a prescribed element, this means that another element can be further included instead of excluding another element.

Hereinafter, an ultraviolet absorber according to the present disclosure, a method for preparing the same, and an ultraviolet blocking product containing the same will be described in detail with reference to example embodiments and drawings. However, the present disclosure is not limited to such example embodiments and drawings.

The ultraviolet absorber according to an example embodiment of the present disclosure includes polymeric carbon nitride having a heterocyclic structure containing C and N.

Since polymeric carbon nitride is cheap and stable, it may solve environmental problems coming therefrom by replacing the use of expensive and unstable conventional organic sunscreens (such as oxybenzone, etc.), and since polymeric carbon nitride has a small amount of highly reactive oxygen species (ROS) generated compared to inorganic sunscreens (titanium dioxide, zinc oxide, etc.) having excellent photocatalytic activities, it may also solve skin troubles caused thereby. In addition, since polymeric carbon nitride has excellent absorption in both UV-A (320-400 nm) and UV-B (290-320 nm) regions and has a thin plate-like two-dimensional structure, it also has good spreadability so that it is easily applied as a sunscreen.

According to an aspect, the heterocyclic structure containing C and N may include one including a triazine structure, a heptazine ring structure, or a combination thereof. That is, since it contains only biologically compatible C, H, and N as chemical constituents, is cheap, stable, and eco-friendly, has biocompatibility, and has a thin plate-like two-dimensional structure, it also has good spreadability so that it is easily applied as a sunscreen.

According to an aspect, polymeric carbon nitride may be contained in an amount of 0.01 to 50% by weight (wt %), more preferably 0.1 to 25 wt % in the ultraviolet absorber (or cosmetic composition).

According to an aspect, the ultraviolet absorber may have a sun protection factor SPF of 3.5 or more, and the ultraviolet absorber may have a sun protection factor (SFA) of 3.0 or more.

Titanium dioxide, which is a typical sunscreen, intensively absorbs only the UV-B region, whereas the polymeric carbon nitride-based ultraviolet absorber according to the present disclosure has a high ultraviolet blocking effect in a wide area over the UV-A and UV-B regions.

As a method of comparing this, it is most common to compare SPF (UV-B) and PFA (UV-A) values. Preferably, when a cream composition including 10 wt % of polymeric carbon nitride is used, the sun protection factors are SPF = 4 and PFA = 3.23 respectively.

In other words, it has excellent absorption for the entire ultraviolet spectra (320-400 nm UV-A + 290-320 nm UV-B wavelength) so that it may be applied to various functional ultraviolet blocking products.

According to an aspect, when the rhodamine B decomposition reaction is performed using the ultraviolet absorber, Equation 1 below may be satisfied.

[Equation 1]

$$C/C_0 > 0.8$$

(However, Co is the initial concentration of rhodamine B, and C is the concentration of rhodamine B remaining after the decomposition reaction.)

That is, since the polymeric carbon nitride-based ultraviolet absorber according to the present disclosure has very low photocatalytic activity and has a small amount of highly reactive oxygen species (ROS) generated compared to inorganic sunscreens (titanium dioxide, zinc oxide, etc.) having excellent photocatalytic activities, it may solve problems that may be caused by ROS.

A method for preparing an ultraviolet absorber according to an example embodiment of the present disclosure includes the steps of: preparing at least one precursor selected from the group consisting of melamine, dicyandiamide, cyanamide, urea, cyanuric acid, and complexes thereof; heating the precursor; drying the heated precursor; acid-treating, heat-treating, or sonicating the dried precursor; and drying the acid-treated, heat-treated or sonicated precursor.

According to an aspect, the step of heating the precursor may be performed at a temperature condition of 400°C to 600°C for 1 to 10 hours. Preferably, the step of heating the precursor may be putting the precursor into a heating furnace (Chamber Furnace UAF, Lenton) and heating it at a temperature condition of 400°C to 600°C for 2 to 5 hours to complete the reaction.

When performing the step of heating the precursor at a temperature condition of less than 400°C, a problem of severely lacking the blocking ability may occur, and when performing the step of heating the precursor at a temperature condition of exceeding 600°C, there may be a problem in that there is little amount that may be obtained after heating due to combustion.

According to an aspect, after performing the step of heating the precursor, a step of cooling the heated precursor, and then washing it with distilled water to remove the residue may be performed.

According to an aspect, the step of drying of the heated precursor may be performed in an oven at a temperature condition of 60°C to 80°C for 8 to 24 hours. Preferably, it may be drying the heated precursor in an oven at a temperature condition of 80°C for 8 hours or more.

When performing the step of drying the heated precursor at a temperature condition of less than 60°C, the drying is not completely performed so that there may be a problem in that it is difficult to check the exact weight, and when performing the step of drying the heated precursor at a temperature condition of exceeding 80°C, damage to the sample may occur and there is a possibility of sample loss due to sudden boiling of water.

According to an aspect, the step of acid-treating the dried precursor may be pouring $H_2SO_4$ (98%), HNO3

(70%), or HCl (35%) into the dried precursor to perform acid treatment, and then pouring distilled water thereinto to generate heat.

According to an aspect, the step of heat-treating the dried precursor may be performed at a temperature condition of 400°C to 600°C for 1 to 2 hours. Preferably, it may be heating the dried precursor once again at a temperature condition of 500°C for 1 to 2 hours.

According to an aspect, the step of sonicating the dried precursor may be pouring isopropyl alcohol (IPA) into the dried precursor to perform sonication for 1 to 6 hours.

According to an aspect, the step of drying the acid-treated, heat-treated, or sonicated precursor may be performed in an oven at a temperature condition of 60°C to 80°C for 8 to 16 hours. Preferably, it may be drying the acid-treated, heat-treated, or sonicated precursor in an oven at a temperature condition of 80°C for 8 hours or more.

When the drying step is performed at a temperature condition of less than 60°C, the drying is not completely performed, and this may cause an increase in the weight of the sample due to residual water.

According to an aspect, the ultraviolet absorber may be an ultraviolet absorber according to an example embodiment of the present disclosure.

An ultraviolet blocking product according to an example embodiment of the present disclosure contains: an ultraviolet absorber according to an example embodiment of the present disclosure; or an ultraviolet absorber prepared through a method for preparing an ultraviolet absorber according to an example embodiment of the present disclosure.

Since polymeric carbon nitride is cheap and stable, it may solve environmental problems coming therefrom by replacing the use of expensive and unstable conventional organic sunscreens (such as oxybenzone, etc.), and since polymeric carbon nitride has a small amount of highly reactive oxygen species (ROS) generated compared to inorganic sunscreens (titanium dioxide, zinc oxide, etc.) having excellent photocatalytic activities, it may also solve skin troubles caused thereby. In addition, since polymeric carbon nitride has excellent absorption in both UV-A (320-400 nm) and UV-B (290-320 nm) regions and has a thin plate-like two-dimensional structure, it also has good spreadability so that it may be used as a sunscreen.

According to an aspect, the ultraviolet blocking product may be an ultraviolet blocking cosmetic product or an ultraviolet blocking filter.

Polymeric carbon nitride-based sunscreens have many promising features including high optical and chemical stabilities and non-allergic properties. Even the nanosheet of a polymeric carbon nitride ultraviolet (UV) filter has better application and good adhesion through the epidermal upper layer (stratum keratin) and does not penetrate easily into the skin. Moreover, it contains only biologically compatible C, H and N as chemical constituents.

Hereinafter, the present disclosure will be described in more detail by Examples and Comparative Example.

However, the following Examples are only for exemplifying the present disclosure, and the contents of the present disclosure are not limited to the following Examples.

Preparation of Polymeric Carbon Nitride (PCN) as Ultraviolet Blocking Cosmetic Product

Polymeric carbon nitride (PCN) was synthesized by heat treatment in air. Melamine, dicyandiamide, cyanamide, urea, etc. were used as a precursor, and the precursor was put into a heating furnace (Chamber Furnace UAF, Lenton) and heated at a temperature condition of 400°C to 600°C for 2 to 5 hours to complete the reaction. After cooling a reaction product to obtain a powder, the powder was washed with distilled water to remove residue, and then dried in an oven at 80°C overnight.

After drying a resulting material and grinding well the dried material, a process of (1) pouring $H_2SO_4$ (98%), HNO3 (70%), or HCl (35%) into the ground material to perform acid treatment, and then pouring distilled water thereto to generate heat, (2) heating again the ground material at 500°C for 1 to 2 hours, or (3) pouring IPA into the ground material to carry out sonication for 1 to 6 hours was performed. After that, a sample was recovered through centrifugation, the sample was washed with distilled water and filtered. The resulting material was dried in an oven at 80°C overnight to obtain PCN.

PCN obtained as described above has a two-dimensional structure in which triazine or heptazine rings composed of C and N heterocycles are repeated.

FIG. 1 is ultraviolet-visible (UV-Vis) absorption spectra of polymeric carbon nitride produced according to an example embodiment of the present disclosure.

Referring to FIG. 1, it can be seen that it has excellent absorption in both UV-A (320-400 nm) and UV-B (290-320 nm) regions when compared with titanium dioxide and zinc oxide which have conventionally been used.

Rhodamine B (RhB) Decomposition Reaction Experiment

Rhodamine B (RhB) decomposition reaction experiment was conducted using polymeric carbon nitride produced according to an example embodiment of the present disclosure and $TiO_2$ and ZnO which have conventionally been used.

The photocatalytic RhB decomposition reaction was performed in a Pyrex reactor, and a 300 W Xe lamp (Xe Arc lamp source, Oriel) having a 1Sun filter (Oriel) mounted thereon was used as a light source. The luminous intensity was measured at 100 mW/cm² using a silicon detector (Peccell Technologies, Japan).

10 mg of a photocatalyst was added to an RhB solution in which 1 mg of RhB had been contained in 100 mL of distilled water, and then dispersed in an ultrasonic vibrator for 10 minutes. In order to confirm the adsorption/desorption equilibrium, the suspension was maintained for 24 hours in the dark under continuous stirring.

After 24 hours, the decomposition of RhB using polymeric carbon nitride, $TiO_2$, and ZnO was started while turning on the light. Including the equilibrium point, 5 mL of each was extracted at 10-minute intervals for 1 hour. In addition, the extracted sample was centrifuged for 20 minutes to settle the powders. The absorbance of the centrifuged solution was collected using UV-3600 (Shimadzu), etc. Further, the photocatalytic activities could be compared through the absorption peak intensity of 552 nm.

FIG. 2 is a graph comparing photocatalytic activities of polymeric carbon nitride produced according to an example embodiment of the present disclosure and conventional titanium dioxide and zinc oxide.

Referring to FIG. 2, it can be seen that polymeric carbon nitride produced according to an example embodiment of the present disclosure has very low photocatalytic activity.

It can be seen through these results that unlike conventional organic sunscreens, which are expensive and also have to be reapplied relatively frequently due to their low photostability, ultraviolet blocking cosmetics to which polymeric carbon nitride produced according to the present disclosure is applied are inexpensive, can be simply produced, and are even stable so that they are very effective in terms of economic feasibility.

Further, it can be seen through that unlike conventional inorganic sunscreens having high photocatalytic activities, ultraviolet blocking cosmetics to which polymeric carbon nitride produced according to the present disclosure is applied have low photocatalytic activities so that they may realize excellent light absorption ability in both UV-A and UV-B regions while making less highly reactive oxygen species.

Although the example embodiments have been described with reference to the limited Examples and drawings as described above, various modifications and variations are possible from the above description by one of ordinary skill in the art. For example, appropriate results can be achieved although described techniques are performed in order different from a described method, and/or described elements are joined or combined in a form different from the described method, or replaced or substituted by other elements or equivalents. Therefore, other example embodiments, other examples, and equivalents to the scope of claims also belong to the scope of the claims to be described later.

**Claims**

1. An ultraviolet absorber comprising polymeric carbon nitride having a heterocyclic structure containing C and N.

2. The ultraviolet absorber of claim 1, wherein the heterocyclic structure containing C and N comprises a triazine structure, a heptazine ring structure, or a combination thereof.

3. The ultraviolet absorber of claim 1, wherein the ultraviolet absorber has a sun protection factor (SPF) of 3.5 or more, and the ultraviolet absorber has a sun protection factor (SFA) of 3.0 or more.

4. The ultraviolet absorber of claim 1, wherein when performing a rhodamine B decomposition reaction using the ultraviolet absorber, Equation 1 below is satisfied:

$$[\text{Equation 1}]$$
$$C/C_0 > 0.8$$

(However, Co is an initial concentration of rhodamine B, and C is a concentration of rhodamine B remaining after the decomposition reaction.).

5. A method for preparing an ultraviolet absorber, the method comprising steps of:

   preparing at least one precursor selected from the group consisting of melamine, dicyandiamide, cyanamide, urea, cyanuric acid, and complexes thereof;
   heating the precursor;
   drying the heated precursor;
   acid-treating, heat-treating, or sonicating the dried precursor; and
   drying the acid-treated, heat-treated or sonicated precursor.

6. The method of claim 5, wherein the step of heating the precursor is performed at a temperature condition of 400°C to 600°C for 1 to 10 hours.

7. The method of claim 5, wherein the step of drying the heated precursor is performed in an oven at a temperature condition of 60°C to 80°C for 8 to 24 hours.

8. The method of claim 5, wherein the step of acid-treating the dried precursor is pouring $H_2SO_4$ (98%), HNO3 (70%), or HCl (35%) into the dried precursor to perform acid treatment, and then pouring distilled water to generate heat.

**EP 3 981 386 A1**

9. The method of claim 5, wherein the step of heat-treating the dried precursor is performed at a temperature condition of 400°C to 600°C for 1 to 2 hours.

10. The method of claim 5, wherein the step of sonicating the dried precursor is pouring isopropyl alcohol (IPA) into the dried precursor to perform sonication for 1 to 6 hours.

11. The method of claim 5, wherein the step of drying the acid-treated, heat-treated, or sonicated precursor is performed in an oven at a temperature condition of 60°C to 80°C for 8 to 16 hours.

12. The method of claim 5, wherein the ultraviolet absorber is the ultraviolet absorber of any one of claims 1 to 4.

13. An ultraviolet blocking product containing the ultraviolet absorber of any one of claims 1 to 4; or an ultraviolet absorber prepared through the method for preparing the ultraviolet absorber of any one of claims 5 to 11.

14. The method of claim 12, wherein the ultraviolet blocking product is an ultraviolet blocking cosmetic product or an ultraviolet blocking filter.

FIG. 1

FIG. 2

<div align="center">

**INTERNATIONAL SEARCH REPORT**

</div>

| | International application No. |
|---|---|
| | **PCT/KR2020/005969** |

| A. | CLASSIFICATION OF SUBJECT MATTER |
|---|---|
| | *A61K 8/49*(2006.01)i; *A61Q 17/04*(2006.01)i |

According to International Patent Classification (IPC) or to both national classification and IPC

| B. | FIELDS SEARCHED |
|---|---|

Minimum documentation searched (classification system followed by classification symbols)
A61K 8/49; B01J 27/24; B01J 35/10; C01B 21/082; C02F 1/30; C09D 7/12; A61Q 17/04

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & keywords: 고분자 카본나이트라이드(polymer carbon nitride), 자외선(UV), 멜라민 (melamine), 디시안디아미드(dicyandiamide), 사이안아마이드(cyanamide), 우레아(urea), 시아누르산(cyanuric acid)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | YUAN, B. et al. UV protection of wood surfaces by graphitic carbon nitride nanosheets. Applied Surface Science. Electronic publication on 30 October 2018. vols. 467-468, pp. 1070-1075. See abstract; pages 1070-1071; and figures 1-8. | 1-14 |
| A | CN 108940338 A (HUNAN UNIVERSITY) 07 December 2018. See claims 1-8. | 1-14 |
| A | CN 108380237 A (LIAONING UNIVERSITY) 10 August 2018. See entire document. | 1-14 |
| A | CN 103785434 A (FUZHOU UNIVERSITY) 14 May 2014. See entire document. | 1-14 |
| A | JP 2017-214235 A (INSTITUTE OF PHYSICAL & CHEMICAL RESEARCH) 07 December 2017. See entire document. | 1-14 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 October 2020** | **26 October 2020** |

| Name and mailing address of the ISA/KR | Authorized officer |
|---|---|
| **Korean Intellectual Property Office** **Government Complex Daejeon Building 4, 189, Cheongsa-ro, Seo-gu, Daejeon, Republic of Korea** **35208** | |
| Facsimile No. **+82-42-481-8578** | Telephone No. |

Form PCT/ISA/210 (second sheet) (July 2019)

INTERNATIONAL SEARCH REPORT
Information on patent family members

International application No.

**PCT/KR2020/005969**

| Patent document cited in search report | | | Publication date (day/month/year) | Patent family member(s) | | | Publication date (day/month/year) |
|---|---|---|---|---|---|---|---|
| CN | 108940338 | A | 07 December 2018 | CN | 108940338 | B | 15 May 2020 |
| CN | 108380237 | A | 10 August 2018 | None | | | |
| CN | 103785434 | A | 14 May 2014 | CN | 103785434 | B | 12 August 2015 |
| JP | 2017-214235 | A | 07 December 2017 | JP | 6722937 | B2 | 15 July 2020 |

Form PCT/ISA/210 (patent family annex) (July 2019)